# EUROPÄISCHE PATENTANMELDUNG

(11) **EP 1 593 351 A2**
(43) Veröffentlichungstag der Anmeldung: **09.11.2005**
(21) Anmeldenummer: 05008987.9
(22) Anmeldetag: 25.04.2005
(51) Int. Cl.: A61C 5/06

(54) **Einzeldosis-Verpackung, besonders für Dentalprodukte**

(30) Priorität: 03.05.2004 DE 102004022046
(71) Anmelder: Heraeus Kulzer GmbH, 63450 Hanau (DE)
(72) Erfinder: Bressler, Christian, 60385 Frankfurt (DE); Tieben, Achim, 63579 Freigericht (DE)
(74) Vertreter: Kühn, Hans-Christian

(57) **Zusammenfassung**

Einzeldosis-Verpackung zur Aufnahme und Applikation von flüssigem oder zähflüssigem Material, umfassend
einen durch Drücken verformbaren Behälter (A) für eine Einzeldosis des Materials,
einen fest mit dem Behälter verbundenen Applikator (C)
eine Ausbringöffnung (E) mit Verschluß (D),
wobei der Applikator in dem Verschluß der Ausbringöffnung angeordnet ist.

## Beschreibung

Die Erfindung betrifft eine Einzeldosis-Verpackung, insbesondere für Dentalprodukte.

Flüssige oder zähflüssigen Dentalmaterialien wie z.B. lichthärtende Dentaladhäsive oder Versiegelungsmaterialien werden häufig in Einzeldosis-Verpackungen, (Single Dose), angeboten, Auf diese Weise sind Sauberkeit und Produktreinheit stets gewährleistet, und die Gefahr der Kontamination ist ausgeschlossen, die beim häufigen Öffnen und Schließen größerer Gebinde besteht

Solche Verpackungen Werden in der Regel mit einem Applikator (Microbrush. Pinsel, Spatel oder Schwamm) appliziert. Dieser muß entweder separat bevorratet werden oder ist in die Single Dose Verpackung integriert In der Regel wird der Applikator mit dem (zäh)flüssigen Material benetzt und das Material z.B. auf einen Zahn auf gebracht. Dazu muß der Flüssigkeitsbehälter in irgendeiner Weise geöffnet werden. Das kann z.B. durch Abdrehen oder Ausdrücken oder durch Durchstoßen einer Membran mit dem Applikator geschehen. Bei der anschließenden Applikation benötigt man beide Hände, d.h, eine Hand hält den Container, die andere den Applikator.

Beispiele für solche Dentalverpackungen sind 3M Espes Prompt L Pop oder Centrix' Lollicaine.

Es wurde gefunden, daß man diese an sich schon bequeme Anwendungsweise überraschenderweise weiter verbessern kann. Ein weiteres Ziel ist die Bereitstellung einer für den Kunden einfach zu handhabenden Einmalanwendung, unter Berücksichtigung der günstiger Kosten und einfacher Produktionsmöglichkeiten.

Dazu wird eine Verpackung zur Verfügung gestellt, bei der das Ausbringen des Materials und die Applikation mit einer Hand möglich sind. Ein flexibler Behälter enthält das flüssige Material. An ihm sind eine Öffnung mit Verschluß und ggf. einem Ausbringkanal, und ein Applikatoraufsatz angeordnet. Der Applikatoraufsatz ist dabei im Verschluß der Behätteröffnung angeordnet. Nach Entfernen des Verschlusses ist der Applikator bereits mit dem flüssigen Material benetzt bzw. wird durch das erstmalige Drücken benetzt, und die Applikation kann beginnen. Durch Druck auf den Behälter kann weiteres Material durch die Öffnung auf den Bürstenaufsatz gedrückt und dann appliziert werden, ohne daß man die Vorrichtung ablegt oder man aus einem zusätzlichen oder separaten Container Produkt nachholen muss.

Die Erfindung betrifft somit eine Einzeldosis-Verpackung zur Aufnahme und Applikation von flüssigem Material gedeckt, die umfaßt:
einen durch Drücken verformbaren Behälter für die Einzeldosis eines flüssigen Materials,
einen fest mit dem Behälter verbundenen Applikator
eine Ausbringöffnung mit Verschluß,
wobei der Applikator in dem Verschluß der Ausbringöffnung angeordnet ist.

Vorteilhaft ist zwischen Behälter Ausbringöffnung ein Ausbringkanal angeordnet, der es erleichtert, das flüssige Material am gewünschten Ort, z.B. an einer Zahnkavität im Mund des Patienten, aufzubringen. Die Anordnung des Applikators direkt im Verschluß gewährleistet, daß der Applikator nach dem Öffnen des Behälters bereits mit dem flüssigen Material benetzt ist oder durch Drücken auf den Behälter benetzt werden kann. Der Ausbringkanal kann beliebig geformt sein oder in seiner Form veränderbar sein. Bewährt ist ein gekrümmtes Rohr oder ein Rohr, das vor der Applikation in die gewünschte Krümmung gebracht werden kann.

Der Behälter kann ein Spritzgußteil sein oder aus Folie bestehen. Zweckmäßig ist eine Kombination von tiefgezogener Folie, die wiederum mit Folie, z.B. Alufolie versiegelt wird. Die Tief ziehfolie kann den Behälter und den Kanal formen. Möglich ist auch eine Kombination von Sprutzgußteilen und Folie, oder eine Kombination miteinander verbindbarer Spritzgußteile. Allerdings ist man bei Spritzgußteilen in der Formgestaltung der Applikationsform relativ eingeschränkt. Aus Folie ist das Material zudem leichter auszudrücken.

Eine bevorzugte Verpackung besteht daher aus zwei Folien, eine davon tiefgezogen. Der Behälter zur Aufnahme des (zäh)flüssigen Materials sowie der Ausbringkanal ist in der Form der tiefgezogenen Folie integriert. Im vorderen Ausbringbereich des Ausbringkanals ist eine Applikationshilfe integriert. Darüber ist eine Verschlusskappe gestülpt. Durch das Öffnen der Verpackung wird nicht nur der Behälter geöffnet, sondern auch die Applikationshilfe in einen funktionsfähigen Zustand gebracht.

Der Applikator kann z.B. ein Microbrush, ein Pinsel, ein Schwamm oder auch die individuell geformte (etwa zum Spatel) Verpackung sein. Der Applikator kann gerade, seitlich, schräg - je nach Anwendungsfall - angebracht werden, oder der Ausbringkanal ist in seiner Form veränderlich.

Vorteile der bevorzugten erfindungsgemäßen Einzeldosis Verpackung aus Folie sind
- Eine Einmal-Anwendung inkl. Applikationshilfe, es brauchen keine Applikatoren vorrätig gehalten zu werden.
- Die Realisierung ist mit wenigen Teilen (2 bis 3 Teile) möglich.
- Bei der Produktiojn entstehen relativ niedrige Kosten für Werkzeuge (im Gegensatz zur Herstellung von Spritzgussteilen).
- Eine Folien(Folie gegen Folie, Folie gegen Spritzgussteil)-Verpackung ist besser drückbar/dosierbar als ein Spritzgussteil.

Eine Ausführungsform der Erfindung wird anhand der Skizzen erläutert.

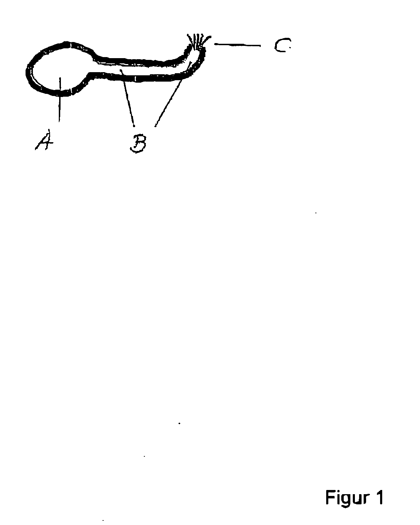

Fig 1 zeigt eine erfindungsgemäße Verpackung mit Behälter A, Öffnung E, Ausbringkanal B und Applikator C, hier gezeigt als Microbrush mit Borsten.

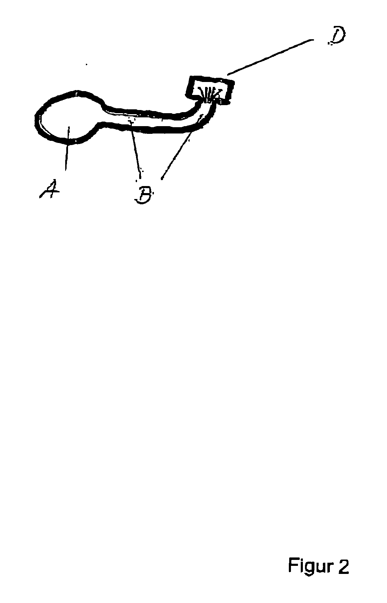

Fig. 2 zeigt die Vorrichtung der Figur 1 mit dem Verschluß D

Nach Entfernen des Verschlusses D, beispielsweise durch Abdrehen oder Abschrauben, kann der benetzte Microbrush sofort für die Applikation eingesetzt werden. Gehalten wird dabei der Behälter, und durch Druck darauf kann weiteres Material auf den Applikator gebracht werden. Die in der Figur gezeigt gekrümmte Form des Ausbringkanals B ermöglicht die Applikation an schwer zugänglichen Orten, etwa in Zahnkavitäten.

## Patentansprüche

1. Einzeldosis-Verpackung zur Aufnahme und Applikation von flüssigem oder zähflüssigem Material, umfassend
einen durch Drücken verformbaren Behälter (A) für eine Einzeldosis des Materials,
einen fest mit dem Behälter verbundenen Applikator (C)
eine Ausbringöffnung (E) mit Verschluß (D),
wobei der Applikator in dem Verschluß der Ausbringöffnung angeordnet ist.

2. Einzeldosis-Verpackung nach Anspruch 1, wobei zwischen Behälter und Ausbringöffnung ein Ausbringkanal (B) angeordnet ist.
